Europäisches Patentamt

European Patent Office

Office européen des brevets

(19)

(11) Veröffentlichungsnummer: **0 313 753**
**A2**

(12) # EUROPÄISCHE PATENTANMELDUNG

(21) Anmeldenummer: 88113765.7

(22) Anmeldetag: 24.08.88

(51) Int. Cl.⁴: **C07D 295/02**

(30) Priorität: 17.10.87 DE 3735212

(43) Veröffentlichungstag der Anmeldung:
03.05.89 Patentblatt 89/18

(84) Benannte Vertragsstaaten:
AT BE CH DE ES FR GB IT LI NL SE

(71) Anmelder: **HÜLS AKTIENGESELLSCHAFT**
**- RSP Patente / PB 15 - Postfach 13 20**
**D-4370 Marl 1(DE)**

(72) Erfinder: **Disteldorf, Josef, Dr.**
**Dahlbrede 47**
**D-4370 Marl(DE)**
Erfinder: **Finke, Norbert, Dr.**
**Untermarkstrasse 59 a**
**D-4600 Dortmund 30(DE)**
Erfinder: **Hübel, Werner, Dr.**
**Keplerstrasse 13**
**D-4350 Recklinghausen(DE)**

(54) **Verfahren zur Herstellung von Gemischen aus 1,4-Diazabicyclo-(2.2.2)-octan und Piperazin.**

(57) Aufgabe der Erfindung ist die Herstellung von Gemischen aus 1,4-Diazabicyclo(2.2.2)-octan und Piperazin aus möglichst einfachen Verbindungen mit möglichst hoher Selektivität.

Dies läßt sich erreichen, indem man Polyamine der Formel $H_2H(CH_2-CH_2-NH)_x-H$ mit x = 1 - 4 und/oder Ethanolamine der Formel $(HO-CH_2-CH_2)_y-NH_{3-y}$ mit y = 1 - 3, mit einem Pentasil bei erhöhter Temperatur in Kontakt bringt.

Die erhaltenen Gemische können direkt zur Herstellung von reinem 1,4-Diazabicyclo(2.2.2)-octan verwendet werden.

EP 0 313 753 A2

## Verfahren zur Herstellung von Gemischen aus 1,4-Diazabicyclo(2.2.2)-octan und Piperazin

Gegenstand der Erfindung ist ein Verfahren zur Herstellung von Gemischen aus 1,4-Diazabicyclo(2.2.2)-octan (auch Triethylendiamin genannt, im folgenden mit "TEDA" abgekürzt) und Piperazin aus Ethanolaminen und Ethylendiamin und seinen Oligomeren wie Diethylentriamin, Triethylentetramin usf.

Aus Gemischen von TEDA und Piperazin können die beiden Bestandteile durch bekannte Aufarbeitungsmethoden isoliert werden. TEDA ist ein technisch bedeutsames Produkt, das u. a. als Katalysator bei der Herstellung von Polyurethanen benötigt wird. Piperazin findet Verwendung als Ausgangsprodukt zur Herstellung von Pharmazeutika. Gemäß Patentanmeldung P 37 35 214.8 der gleichen Anmelderin vom gleichen Tage "Verfahren zur Herstellung von 1,4-Diazabicyclo(2.2.2)-octan aus Piperazin" ist es auch möglich, die Gemische als Ausgangsprodukt für die Herstellung von TEDA einzusetzen.

Die Verfahren zur Herstellung von TEDA unterscheiden sich im wesentlichen durch die Art der Ausgangsprodukte und der Katalysatoren.

Grundsätzlich wäre es am günstigsten, wenn man bei der Herstellung von TEDA von möglichst einfachen Verbindungen ausgehen könnte. Dem Einsatz der als Ausgangsverbindungen vielfach verwendeten Basischemikalien Ethanolamin und Ethylendiamin und deren Oligomeren steht entgegen, daß die bekannten Verfahren - seien sie phosphat- oder alumosilikatkatalysiert - nur eine sehr geringe Selektivität aufweisen.

So ist aus der US-PS 2 937 176 ein Verfahren bekannt, bei dem man Diethylentriamin bei etwa 360 °C und atmosphärischem Druck über ein Alumosilikat mit 86 % $SiO_2$ und 12 % $Al_2O_3$ mit einer Raumgeschwindigkeit von 1,2 Volumenteilen Einsatzprodukt pro Volumenteil Katalysator leitet. Das nach Abtrennung von unumgesetztem Diethylentriamin erhaltene Reaktionsgemisch enthält nur etwa 11 % Piperazin und 10 % TEDA.

Die DE-OS 24 34 913 beansprucht gemäß Anspruch 2 ein Verfahren zur Herstellung von heterocyclischen Verbindungen wie TEDA und Piperazin durch Cyclisierung von Ethanolaminen als auch von Ethylendiamin, Diethylentriamin und dessen höheren Homologen an einem Zeolithen der Formel

$$a(M_{2/n}O) \bullet (Al_2O_3) \bullet m(SiO_2),$$

wobei a den Wert 1,0 ± 0,5 hat, M ein Kation eines (Erd)Alkalimetalles, eines Elementes der Zinkgruppe, ein Proton oder ein Ammoniumion ist, n dessen Valenz bedeutet und m, das Verhältnis von $SiO_2$ zu $Al_2O_3$, einen Wert von 2 bis 12 hat. Der Umsetzungsgrad bei Einsatz von Ethanolaminen liegt über 80 %, der von Ethylendiamin dagegen nur bei 20 %. Die Selektivitäten sind generell schlecht; im Falle von Ethanolamin als Ausgangsprodukt betragen sie für TEDA 15 % und für Piperazin 13 %. Ein bedeutsames Nebenprodukt ist in diesem Falle Morpholin.

Das in der US-PS 3 285 920 beschriebene Verfahren zur gleichzeitigen Herstellung von TEDA und Piperazin ist ein 2-Stufenprozess, bei dem man zunächst Ethylendiamin, Ethanolamin und/oder deren Oligomere in Gegenwart von Ammoniak, Wasserstoff und eines Hydrierkatalysators zu einem Gemisch aus Piperazin und N-(Beta-aminoethyl)-piperazin umsetzt und den verbleibenden Rest - nach Abtrennung des Piperazins - in Gegenwart der bekannten Phosphat- und Alumosilikatkatalysatoren cyclisiert. Die Ausbeuten an TEDA liegen bei etwa 20 %, die von Piperazin bei etwa 12 %.

Die US-PS 3 369 019 offenbart gemäß Anspruch 18 u. a. ein Verfahren zur Herstellung von TEDA, bei dem man Ethanolamin mit Ammoniak bei Temperaturen von 100 bis 400 °C an einem mit Seltenen Erden dotiertem Faujasit umsetzt. Die gaschromatographisch bestimmte Ausbeute an TEDA beträgt laut Beispiel 15 nur 20 %. Daneben entstehen eine Vielzahl von kondensierten Aminen.

Den Verfahren nach dem Stand der Technik ist also eine geringe Selektivität im Hinblick auf die Bildung der beiden gewünschten Produkte TEDA und Piperazin gemeinsam.

Ziel der vorliegenden Erfindung war es, ein Verfahren zur Herstellung von Gemischen aus TEDA und Piperazin aus leicht zugänglichen N-haltigen Ausgangsverbindungen zu entwickeln, das vor allem eine höhere Selektivität gewährleisten sollte.

Es wurde jetzt ein solches Verfahren gefunden, bei dem man als N-haltige Ausgangsverbindung Polyamine der Formel
$H_2N-(CH_2-CH_2-NH)_x-H$
mit x = 1 - 4
und/oder Ethanolamine der Formel
$(HO-CH_2-CH_2)_y-NH_{3-y}$
mit y = 1 - 3

und als Katalysator einen Pentasil der allgemeinen Formel
$(M^I \bullet M^{II}O_2) \bullet aM^{III}O_2 \bullet bH_2O$ einsetzt. Hierbei bedeutet

$M^I$ ein Äquivalent eines 1-einwertigen Alkalimetallkations, ein Proton, die Ammoniumgruppe oder ein halbes Äquivalent eines 2-wertigen Erdalkalimetallkations,

$M^{II}$ ein Äquivalent eines der folgenden 3-wertigen Kationen $Al^{3+}$, $B^{3+}$, $Sb^{3+}$, $As^{3+}$, $Cr^{3+}$, $V^{3+}$, $Ga^{3+}$ und $Fe^{3+}$

$M^{III}$ ein Äquivalent eines der folgenden 4-wertigen Kationen $Si^{4+}$, $Ge^{4+}$, $Ti^{4+}$ und $Zr^{4+}$

a im Falle von $M^{II}$ = $Al^{3+}$ und $M^{III}$ = $Si^{4+}$ einen Wert größer als 6 und sonst mindestens 1,
und der Wert von

b nach oben hin lediglich durch die Forderung begrenzt ist, daß eine Zeolith-Struktur aufrecht erhalten bleiben muß.

Vorzugsweise handelt es sich um einen Pentasil des ZSM 5-Typs. Das Verfahren wird vorzugsweise so durchgeführt, daß man die Ausgangsprodukte bei einem Absolutdruck von 0,1 bis 10 bar in gasförmiger Form über einen Festbettkatalysator leitet, eine Temperatur von 250 bis 550 °C, insbesondere 280 bis 380 °C, einstellt und die Durchflußgeschwindigkeit so einstellt, daß die Katalysatorbelastung (LHSV) 0,1 bis 10 Stunden⁻¹ beträgt.

Das erfindungsgemäße Verfahren zeichnet sich dadurch aus, daß man von einfachen, preisgünstigen, leicht zugänglichen Ausgangsprodukten ausgehen kann und in einem Schritt zwei technisch interessante, strukturell anspruchsvolle heterocyclische Verbindungen erhält.

Als N-haltige Ausgangsverbindungen eignen sich die Polyamine der Formel $H_2N\text{-}(CH_2\text{-}CH_2\text{-}NH)_x\text{-}H$
mit x = 1 - 4, insbesondere Ethylendiamin,
sowie die Ethanolamine der Formel $(HO\text{-}CH_2\text{-}CH_2)_y\text{-}NH_{3\text{-}y}$
mit y = 1 - 3, insbesondere Ethanolamin.

Für das erfindungsgemäße Verfahren eignen sich alle Pentasile der oben aufgeführten Formel mit den angegebenen Bedeutungen von $M^I$, $M^{II}$, $M^{III}$, a und b. Vorzugsweise hat a einen Wert zwischen 15 und 200 und b einen Wert zwischen 0 und 8. Typischerweise steht $M^{II}$ für $Al^{3+}$ und $M^{III}$ für $Si^{4+}$. Ersetzt man eines dieser beiden Metalle im Sinne einer sogenannten isomorphen Substitution durch andere Metalle, wie sie oben angegeben sind, so erhält man Pentasile, deren Kristallstruktur im wesentlichen unverändert ist (vgl. Chem.-Ing. Techn. 58, 946 - 959 und 969 -971 (1986). Es ist auch möglich, sowohl Aluminium als auch Silizium zu ersetzen. Es besteht auch die Möglichkeit der teilweisen Substitution. Die Pentasile können auch mit Übergangsmetallen und/oder Seltenen Erden dotiert werden. Zur Erhöhung der Standfestigkeit des Pentasils werden diese vielfach mit anderen Materialien wie z. B. Cellulosematerialien, Tonen, Metalloxiden oder Bindemitteln versetzt und erhalten so ihre Form.

Die erfindungsgemäß verwendbaren Pentasile und deren Herstellung werden beispielsweise in Ullmanns Encyclopädie der Technischen Chemie, 4. Auflage, Band 24, Seite 575 (1983) und Band 17, Seite 9 (1979) beschrieben.

Bevorzugte Pentasile werden in der DE-OS 32 39 054 beschrieben. Insbesondere werden Pentasile mit ZSM 5-Struktur verwendet, wie sie in der obengenannten Monographie aufgeführt sind.

Es ist zweckmäßig, die Ausgangsverbindungen, gegebenenfalls zusammen mit einem Verdünnungsmittel, wie z. B. Wasser, einem inerten Kohlen wasserstoff, der bei Raumtemperatur flüssig ist, oder einem Gas, wie z. B. Stickstoff oder Ammoniak, dem Katalysator zuzuführen. Dabei geht man üblicherweise so vor, daß man die flüssigen Ausgangsverbindungen zusammen mit der gasförmigen Komponente in einer geeigneten Aufwärmvorrichtung auf eine Temperatur aufheizt, bei der alle Komponenten im gasförmigen Zustand vorliegen. Man kann die Ausgangsverbindungen auch in flüssigen Kohlenwasserstoffen lösen und entsprechend verfahren.

Der Druck ist keine kritische Größe des vorliegenden Verfahrens, obwohl dem Fachmann bekannt ist, daß der Umsatz mit steigendem Druck zunimmt. Im allgemeinen sollte der Druck zwischen 0,1 und 50 bar liegen.

Das Verfahren besteht grundsätzlich darin, daß die Ethanolamine und/oder Polyamine mit dem Zeolithen in Kontakt gebracht werden. Es ist üblich, die gasförmigen Ausgangsverbindungen in einem Rohr über den fest angeordneten Zeolithen zu leiten. Die Reaktionstemperatur beträgt 250 bis 550 °C, vorzugsweise 280 bis 380 °C . Die Katalysatorbelastung wird vorzugsweise so eingestellt, daß die Katalysatorbelastung (LHSV, liquid hourly space velocity) 0,1 bis 10 Stunden⁻¹ beträgt (siehe Ullmanns Encyklopädie der technischen Chemie, 4. Auflage, Band 13, S. 550, Verlag Chemie, Weinheim, 1977). Es hat sich als vorteilhaft erwiesen, alle produktführenden Leitungen, Pumpen und Abscheider auf eine Temperatur von 40 bis 80 °C aufzuheizen.

Die Aufarbeitung des Reaktionsgemisches kann nach üblichen Methoden erfolgen. Eine besonders zweckmäßige Form besteht darin, das Reaktionsgemisch aufzufangen und gemäß Patentanmeldung P 37 35 214.8 der gleichen Anmelderin vom gleichen Tage "Verfahren zur Herstellung von 1,4-Diazabicyclo-(2.2.2)-octan aus Piperazin" weitgehend vollständig zu TEDA umzusetzen. Eine andere Möglichkeit besteht darin, aus dem Reaktionsgemisch die beiden Komponenten rein zu isolieren.

## Beschreibung der Versuchsapparatur

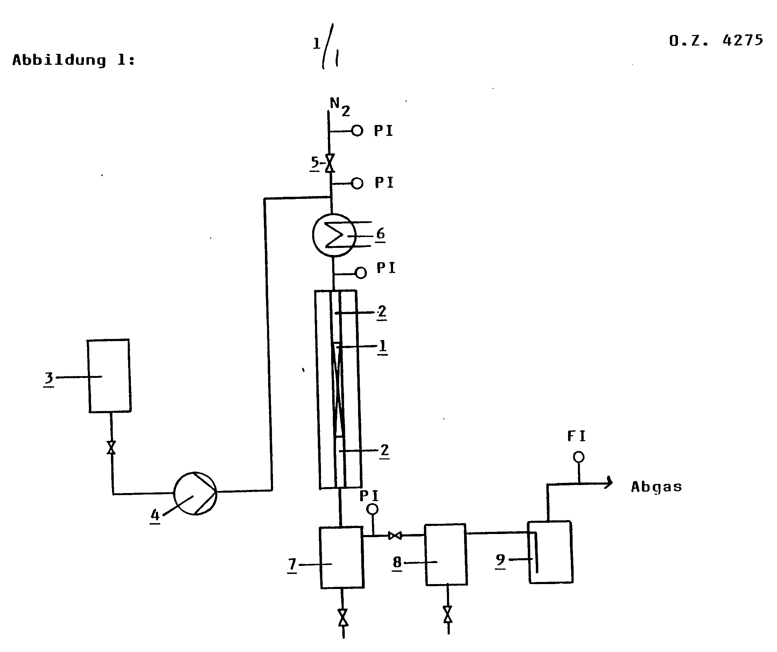

Die Versuche werden in einem Festbett-Rohrreaktor aus Edelstahl (Länge 1350 mm, Durchmesser 22 mm) mit Sandwirbelbettbeheizung und einem innenliegenden Temperaturmeßrohr (Durchmesser 6 mm) durchgeführt. In Abbildung 1 ist der Versuchsaufbau schematisch dargestellt. In der Mitte des Reaktors befindet sich eine 350 mm lange Kontaktzone (1), die mit 120 ml Katalysator gefüllt ist und an die sich oben und unten zwei 500 mm lange Füllkörperschichten (2) anschließen. Das Einsatzprodukt wird aus der kalibrierten Vorlage (3) über eine Membranpumpe (4) dosiert und zusammen mit einem Stickstoffstrom, der über ein Reduzierventil (5) geregelt wird, in dem Wärmeaustauscher (6) auf 200 °C aufgeheizt und von oben in den Reaktor geleitet. Die Reaktionsprodukte werden in zwei hinter einander geschalteten Abscheidern (7 und 8) und einer mit Wasser gefüllten Waschflasche (9) aufgefangen; der Abgasstrom wird über ein Ventil und eine Gasuhr (FI) geregelt. Die Aufarbeitung der Austrittsprodukte erfolgt durch Destillation in einer Füllkörperkolonne (Länge 1700 mm, Durchmesser 30 mm, gefüllt mit V4A-Maschendrahtringen 4 x 4 mm) mit thermostatisierbarem Kolonnenkopf. Der Destillationsschnitt mit einer Siedetemperatur von 140 bis 180 °C ist das gewünschte Substanzgemisch. Durch eine erneute Feindestillation in der gleichen Apparatur können TEDA und Piperazin getrennt werden. Durch Umkristallisation aus Ethylacetat erhält man farbloses TEDA in einer Reinheit von mehr als 99,5 %.

In den folgenden Beispielen Nr. 1, 2, 4, 5, 7 und 8 ist der mit A bezeichnete Katalysator ein Pentasil in der H-Form nach Beispiel 1 der DE-OS 32 39 054 (molares Verhältnis $SiO_2/Al_2O_3$ = 30,8), der zur Konfektionierung mit 40 Gew.-% eines inerten Bindemittels ausgeformt wurde. Der mit B bezeichnete Katalysator entspricht Beispiel 4 der DE-OS 32 39 054 (molares Verhältnis $SiO_2/Al_2O_3$ = 90,0), ebenfalls mit 40 Gew.-% eines inerten Bindemittels konfektioniert.

## Beispiele 1 bis 3

Als Einsatzprodukt wird eine 40 %ige wäßrige Lösung von Ethylendiamin eingesetzt. Der Einfluß der Reaktionstemperatur und des Katalysators auf die Reaktion ist aus der Tabelle zu ersehen.

## Beispiel 4 bis 6

In den Beispielen 4 bis 6 wird Diethylentriamin als Einsatzprodukt verwendet (siehe Tabelle). Das als Nebenprodukt entstehende Ethylendiamin (EDA) kann nach den Beispielen 1 bis 3 zu Gemischen aus TEDA und Piperazin umgesetzt werden.

## Beispiel 7 bis 9

Der Einsatz von 2-Aminoethanol (siehe Tabelle) führt unter vergleichbaren Reaktionsbedingungen zu geringeren Umsätzen als die Verwendung von EDA oder Diethylentriamin (DETA).

Tabelle

| | | | | | | Reaktionsprodukt | | | | | | Selektivität | |
|---|---|---|---|---|---|---|---|---|---|---|---|---|---|
| Beisp. Nr. | Kat. | Temp. °C | Einsatzmenge ml/h | Abgas l/h | Druck (abs.) bar | TEDA % | PIP % | EDA % | MEA % | DETA % | Umsatz % | (TEDA) % | (PIP) % |
| 1 | A | 270 | 100 | 100 | 3 | 6,12 | 16,08 | 77,26 | - | - | 23 | 27 | 71 |
| 2 | A | 330 | 100 | 100 | 3 | 22,11 | 36,95 | 35,20 | - | - | 65 | 34 | 57 |
| 3 | B | 330 | 100 | 100 | 3 | 27,55 | 36,87 | 29,94 | - | - | 70 | 39 | 53 |
| 4 | A | 320 | 100 | 200 | 2 | 26,88 | 24,91 | 17,51 | - | 22,75 | 77 | 35 | 32 |
| 5 | A | 350 | 100 | 50 | 2 | 36,81 | 23,69 | 18,74 | - | 0,66 | 99 | 37 | 24 |
| 6 | B | 330 | 100 | 100 | 3 | 18,15 | 17,88 | 28,86 | - | 16,71 | 83 | 22 | 21 |
| 7 | A | 300 | 100 | 50 | 2 | 5,69 | 4,70 | 0,90 | 87,65 | - | 12 | 46 | 38 |
| 8 | A | 320 | 100 | 200 | 2 | 9,65 | 8,77 | 1,43 | 77,18 | - | 23 | 42 | 38 |
| 9 | B | 330 | 100 | 100 | 3 | 14,21 | 13,69 | 2,17 | 63,05 | - | 37 | 38 | 37 |

**Herstellung von TEDA und Piperazin**

**Erläuterungen zur Tabelle:**

Zusammensetzung lt. gaschromatographischer Bestimmung nach Abzug von Wasser und Ammoniak

Selektivität = Massenanteil TEDA bzw. Pip im Reaktionsprodukt nach Abzug von Ammoniak und Wasser, bezogen auf umgesetztes Einsatzprodukt

Das Abgas besteht im wesentlichen aus dem zugeführten Schleppgas (Stickstoff) und einem geringen Anteil von Ammoniak.

Verwendete Abkürzungen:

EDA = Ethylendiamin

DETA = Diethylentriamin

MEA = 2-Aminoethanol

PIP = Piperazin

EP 0 313 753 A2

**Ansprüche**

1. Verfahren zur Herstellung von Gemischen aus 1,4-Diazabicyclo(2.2.2)-octan und Piperazin aus leicht zugänglichen N-haltigen Ausgangsverbindungen durch In-Kontakt-Bringen der gasförmigen Ausgangsverbindungen mit Feststoffen mit Zeolith-Struktur bei erhöhter Temperatur und anschließende Destillation, dadurch gekennzeichnet,

daß man

- als N-haltige Ausgangsverbindung Polyamine der Formel

$H_2N-(CH_2-CH_2-NH)_x-H$ mit x = 1 - 4

und/oder Ethanolamine der Formel

$(HO-CH_2-CH_2)_y-NH_{3-y}$ mit y = 1 - 3 und man

- als Feststoff einen Pentasil der allgemeinen Formel

$(M^I \bullet M^{II}O_2) \bullet aM^{III}O_2 \bullet bH_2O$

einsetzt, wobei

$M^I$ ein Äquivalent eines einwertigen Alkalimetallkations, ein Proton, die Ammoniumgruppe oder ein halbes Äquivalent eines 2-wertigen Erdalkalimetallkations ist,

$M^{II}$ ein Äquivalent eines der folgenden 3-wertigen Kationen ist

$Al^{3+}$, $B^{3+}$, $Sb^{3+}$, $As^{3+}$, $Cr^{3+}$, $V^{3+}$, $Ga^{3+}$ und $Fe^{3+}$

$M^{III}$ ein Äquivalent eines der folgenden 4-wertigen Kationen ist

$Si^{4+}$, $Ge^{4+}$, $Ti^{4+}$ und $Zr^{4+}$

a im Falle von $M^{II} = Al^{3+}$ und $M^{III} = Si^{4+}$ einen Wert größer als 6 aufweist und sonst mindestens den Wert 1 hat,

und der Wert von

b nach oben hin lediglich durch die Forderung begrenzt ist, daß eine Zeolith-Struktur aufrecht erhalten bleiben muß.

2. Verfahren nach Anspruch 1,

dadurch gekennzeichnet,

daß

$M^I$ ein Proton,

$M^{II}$ $B^{3+}$ oder vorzugsweise $Al^{3+}$ und

$M^{III}$ $Ge^{4+}$ oder vorzugsweise $Si^{4+}$ ist.

3. Verfahren gemäß Anspruch 1 oder 2,

dadurch gekennzeichnet,

daß man als Feststoff einen Pentasil vom Typ ZSM 5 einsetzt.

4. Verfahren gemäß den Ansprüchen 1 bis 3,

dadurch gekennzeichnet,

daß man die Einsatzprodukte in gasförmiger Form über einen Festbettkatalysator bei einem Absolutdruck von 0,1 bis 10 bar leitet.

5. Verfahren gemäß den Ansprüchen 1 bis 4,

dadurch gekennzeichnet,

daß man die Reaktion bei einer Temperatur von 250 bis 550 °C durchführt.

6. Verfahren gemäß Anspruch 5,

dadurch gekennzeichnet,

daß man eine Katalysatorbelastung (LHSV) von 0,1 bis 10 pro Stunde einstellt.

Abbildung 1:

EP 0 313 753 A2